Europäisches Patentamt

European Patent Office

Office européen des brevets

(11) Numéro de publication: **0 268 525 B1**

## (12) FASCICULE DE BREVET EUROPEEN

(45) Date de publication de fascicule du brevet:
19.06.91

(51) Int. Cl.5: **B01J 23/36**, B01J 37/08, C07C 6/04

(21) Numéro de dépôt: 87402491.2

(22) Date de dépôt: 04.11.87

(54) Procédé de préparation d'un catalyseur renfermant du rhenium, catalyseur obtenu et utilisation de ce catalyseur pour la production d'oléfines par métathèse.

(30) Priorité: 18.11.86 FR 8616129

(43) Date de publication de la demande:
25.05.88 Bulletin 88/21

(45) Mention de la délivrance du brevet:
19.06.91 Bulletin 91/25

(84) Etats contractants désignés:
BE DE ES GB IT NL

(56) Documents cités:
FR-A- 1 474 593
FR-A- 2 521 872
US-A- 3 641 189
US-A- 4 006 102

INDUSTRIAL AND ENGINEERING CHEMISTRY,
vol. 60, no. 11, novembre 1968, pages 10-19,
Columbus, Ohio, US; W.H. DAVENPORT et al.:
"Advances in rhenium catalysts"

(73) Titulaire: **INSTITUT FRANCAIS DU PETROLE**
**4, Avenue de Bois-Préau**
**F-92502 Rueil-Malmaison(FR)**

(72) Inventeur: **Chauvin, Yves**
**64, avenue du Géné ral Leclerc**
**F-78230 Le Pecq(FR)**
Inventeur: **Commereuc, Dominique**
**32, rue Abel Vacher**
**F-92190 Meudon(FR)**
Inventeur: **Hugues, François**
**10, Rue du Clos Chaland**
**F-69390 Charly(FR)**
Inventeur: **Saussine Lucien**
**2, Place des Freres Tissandier**
**F - 78290 Croissy sur Seine(FR)**

## Description

L'invention concerne un procédé de préparation d'un catalyseur renfermant du rhénium, le catalyseur obtenu et l'utilisation de ce catalyseur pour la production d'oléfines par métathèse.

La métathèse des oléfines ou réaction de redistribution des groupements alkylidènes entre eux, présente un grand intérêt pratique, par exemple pour le rééquilibrage entre elles des oléfines légères issues du steam-cracking : éthylène, propylène et butènes.

On connaît différents types de catalyseurs susceptibles de catalyser cette réaction, soit de type homogène, quand leurs éléments constitutifs sont tous solubles dans le milieu de la réaction, soit de type hétérogène, quand au moins un des éléments est insoluble dans le dit milieu. Ces derniers sont particulièrement intéressants quand le métal actif est onéreux et qu'il est nécessaire d'envisager sa réutilisation sans pertes. C'est le cas des catalyseurs à base de rhénium dont l'emploi sous forme hétérogène a été préconisé pour catalyser la métathèse des oléfines simples, par exemple dans US 3 641 189 et US 3 676 520.

L'invention décrit un procédé permettant d'obtenir des catalyseurs plus actifs que ceux de l'art antérieur.

Le procédé comporte : (a) une imprégnation d'un support à base d'alumine par une solution d'un composé de rhénium, (b) un chauffage subséquent à 85-250°C au cours duquel le solvant d'imprégnation est éliminé et (c) un chauffage final à 400-1000°C, destiné à activer le catalyseur.

Le procédé est caractérisé en ce que l'imprégnation du support est une imprégnation à sec, aussi appelée imprégnation capillaire, et en ce que l'on maintient le support imprégné, après la fin de l'addition de la solution du composé de rhénium, et avant le chauffage à 85-250°C, pendant au moins dix heures à une température de 0-80°C dans des conditions substantiellement non-évaporantes, c'est à dire telles que au moins 85 % et de préférence au moins 95 % du solvant adsorbé demeure dans le support imprégné. La technique ici se differencie donc du celle décrite dans FR-A-2521872 où ce haitememt pendant au moins 10 heures n'est pas effectue

Par imprégnation à sec, on entend une imprégnation par un volume de solution inférieur ou au maximum égal au volume des pores du support ; de préférence, le volume de solution est au moins 90 % du volume des pores du support.

Le support est, de préférence, l'alumine ou un mélange d'au moins 20 % en poids d'alumine avec un autre oxyde réfractaire, par l'exemple la silice, la magnésie ou l'oxyde de titane. Ce support doit présenter une surface appréciable, par exemple au moins 10 m² /g, et un volume de pores appréciable, par exemple au moins 0,1 cm³/g, de préférence 0,3-1 cm³/g.

Ce volume minimal est un volume libre de liquide, sinon l'imprégnation ne pourrait se faire. Peu importe, par contre, la nature du gaz qui occupe les pores. Ce gaz peut également avoir été chassé par mise sous vide.

Les composés du rhénium préférés sont l'heptoxyde de rhénium, le perrhénate d'ammonium et l'acide perrhénique. Le composé du rhénium est mis en solution dans l'eau ou dans un solvant organique, par exemple un alcool tel que le méthanol. En plus du rhénium on peut introduire d'autres métaux dans le catalyseur. La littérature et les brevets en donnent de nombreux exemples et il est donc inutile d'en donner une liste exhaustive ici.

Le support imprégné est maintenu à 0-80°C pendant au moins dix heures, par exemple 10 à 1000 heures dans les conditions indiquées plus haut. Une durée d'imprégnation largement supérieure à dix heures n'apporte généralement pas d'avantages supplémentaires. Au contraire, une durée d'imprégnation et de maintien à 0-80°C plus brève, par exemple de dix minutes à deux heures, conduit à des catalyseurs moins actifs. Ce fait est surprenant car il est généralement admis que l'imprégnation à sec nécessite un temps beaucoup plus court, de l'ordre de quelques minutes, voir par exemple : A.V. NEIMA RK, L.I. KHEIFEZ, et V.B. FENELONOV dans : Ind. Eng. Chem. Prod. Res. Dev., (1981) 20, 439-450, en particulier page 441.

Le chauffage subséquent (étape b) a pour objet d'éliminer au moins la majeure partie du solvant, et de préférence la quasi-totalité de ce dernier, et de stabiliser le rhénium. Un chauffage à une température de, par exemple 85 à 250°C, de préférence 100 à 180°C, pendant, par exemple, trente minutes à cinq heures ou plus, donne des résultats satisfaisants. On peut opérer en atmosphère neutre, par exemple par balayage d'azote, ou oxydante, par exemple par balayage d'air, ou encore sous-vide. Une durée de chauffage trop courte ou une température trop basse est à éviter car elle conduit à une stabilisation incomplète et à une perte de rhénium au cours du traitement ultérieur d'activation ; inversement, une température trop élevée conduit à des pertes de rhénium au cours de la présente étape.

On règle la quantité de rhénium sur le support par le choix de la concentration de la solution d'imprégnation contenant le rhénium. Lorsque la quantité de rhénium que l'on désire imprégner est supérieure à celle que permet d'introduire une solution à sa limite de saturation, on doit effectuer l'opération en plusieurs fois, en répétant à chaque fois la suite des étapes (a) et (b) jusqu'à obtention de la teneur en rhénium recherchée sur le support.

Ceci permet d'obtenir un solide contenant de 0,01 à 20 % et de préférence de 1 à 15 % en poids sec de rhénium.

L'activation du catalyseur obtenu à la suite des étapes (a) et (b) s'effectue par chauffage entre 400 et 1000 °C (étape c), de préférence entre 500 et 900 °C. Ce chauffage se fait sous une atmosphère de gaz non réducteur, par exemple, oxygène, azote ou argon, oxygène dilué par de l'azote, de préférence sous air, sous conditions statiques ou dynamiques, un léger courant gazeux étant cependant préférable. Le taux d'humidité du courant gazeux est de préférence maintenu inférieur à 200 ppm (parties par million). On peut aussi effectuer le chauffage en atmosphère constituée par les gaz de combustion du méthane en présence d'un excès d'air. La durée de ce traitement d'activation est par exemple de dix minutes à cinq heures ou davantage, après quoi le catalyseur actif ainsi obtenu est refroidi sous atmosphère de préférence anhydre. On peut avantageusement procéder à une purge à l'azote, si nécessaire, avant mise en contact avec la charge hydrocarbonée.

Les oléfines susceptibles de réagir en métathèse catalysée par le catalyseur de rhénium supporté précédemment décrit peuvent être des oléfines linéaires répondant à la formule générale :
$R_1R_2C = CR_3R_4$ ; où $R_1$, $R_2$, $R_3$ et $R_4$, identiques ou différents, sont l'hydrogène ou un radical hydrocarbyle de 1 à 20 atomes de carbone. Les oléfines peuvent aussi être de structure cyclique, le cycle comportant de 3 à 20 atomes de carbone. On peut soit faire réagir une oléfine sur elle-même, soit faire réagir plusieurs oléfines entre elles en mélange. Un exemple d'application est la production de propylène par réaction de l'éthylène avec les butènes-2, ou la réaction inverse de transformation du propylène en un mélange éthylène + butènes-2.

La réaction de métathèse se fait de préférence en l'absence de solvant. Cependant, la présence d'un solvant tel qu'un hydrocarbure, ou un hydrocarbure halogéné, aliphatique, cyclanique ou aromatique, n'est pas néfaste.

La réaction peut être effectuée dans un réacteur agité, ou en faisant passer le ou les réactifs au travers d'un lit fixe, mobile ou fluidisé, de catalyseur.

On opère le plus souvent à une température comprise entre 0 et 200 °C, de préférence entre 20 et 120 °C. La pression n'est pas critique.

La réaction de métathèse est réalisée de préférence en phase liquide, en l'absence d'oxygène et d'humidité, et les réactifs et solvants sont préalablement utilement traités en ce sens.

Les exemples suivants illustrent l'invention sans en limiter la portée.

**EXEMPLE 1**

**PREPARATION DU CATALYSEUR :**

Trente grammes d'une alumine-$\gamma$ (surface spécifique : 187 m²/g, volume poreux : 0,52 cm³/g) sont calcinés sous courant d'air à 350 °C pendant trois heures. Après refroidissement à température ambiante, cette alumine est imprégnée par une solution aqueuse de 1,66 g. de perrhénate d'ammonium (correspondant à une teneur en rhénium théorique du catalyseur de 3,85 % en poids) dans 15,6 cm³ d'eau, en introduisant goutte à goutte la solution aqueuse sur l'alumine et en agitant vigoureusement. Lorsque toute la solution aqueuse a été absorbée par l'alumine, ce qui prend environ cinq minutes, on laisse celle-ci reposer pendant 24 heures à 20 °C en atmosphère humide. Au bout de ce laps de temps, le solide, dont le poids n'a pas varié sensiblement, et porté en 10 minutes, au moyen d'un bain d'huile extérieur, à une température de 140 °C sous une pression de 0,1 mmHg et maintenu dans ces conditions pendant deux heures. A ce moment, le catalyseur est remis sous pression atmosphérique d'air, puis balayé par un courant d'air contenant 150 ppm d'eau avec un débit de 18 l/h. La température est progressivement augmentée jusqu'à 800 °C, niveau que l'on maintient pendant trois heures. Le catalyseur ainsi activé est refroidi sous courant d'air jusqu'à température ambiante, puis purgé plusieurs fois à l'azote et stocké sous azote.

**Utilisation en méthathèse :**

Dix sept grammes du catalyseur préparé ci-dessus sont chargés à l'abri de l'humidité dans un réacteur constitué par un tube à double enveloppe avec circulation d'eau permettant la régulation de la température. Du propylène liquide est injecté au moyen d'une pompe, sur le catalyseur, par le bas du réacteur, avec un débit de 107 cm³/h (débit en volume/volume de catalyseur x heure : VVH = 4,40). La température est de 35 °C et la pression dans le réacteur est maintenue à 3,5 MPa au moyen d'un régulateur placé en aval du réacteur. Dans ces conditions, la conversion du propylène à la sortie du réacteur est de 20 %, en un mélange équimolaire d'éthylène et de butènes-2. La sélectivité est pratiquement de 100 %.

**EXEMPLE 2**

**PREPARATION DU CATALYSEUR :**

Un nouveau lot de catalyseur est préparé comme dans l'exemple 1, à cette différence près que, après l'imprégnation par la solution aqueuse de perrhénate d'ammonium, on laisse reposer le catalyseur imprégné pendant quinze jours à température ambiante, en atmosphère humide, avant de le soumettre ensuite aux traitements thermiques comme décrits dans l'exemple 1.

**Utilisation en métathèse :**

Dans le même appareillage que celui décrit dans l'exemple 1, on charge 17 grammes du catalyseur préparé ci-dessus. Du propylène liquide est injecté avec un débit de 114,3 cm³/h (VVH = 4,70), à une température de 35°C et sous une pression de 3,5 MPa. La conversion du propylène est de 18 % (sélectivité : pratiquement 100 %)

**EXEMPLE 3 (Comparatif)**

Cet exemple ne fait pas partie de l'invention mais est donné à titre de comparaison.

**PREPARATION DU CATALYSEUR:**

Un lot de catalyseur est préparé comme dans l'exemple 1, à cette différence près que après l'imprégnation par la solution aqueuse de perrhénate d'ammonium, ou laisse reposer le catalyseur imprégné seulement quinze minutes avant de le soumettre aux traitements thermiques comme décrits dans l'exemple 1. On opère donc ici conformément à la technique décrite dans FR-A-2521872.

**Utilisation en métathèse :**

Dans le même appareillage que celui décrit dans l'exemple 1, on chargé 17 grammes du catalyseur préparé ci-dessus. Du propylène liquide est injecté avec un débit de 107 cm³/h (VVH = 4,40), à une température de 35°C et sous une pression de 3,5 MPa. La conversion du propylène est de 10 %. La sélectivité est pratiquement de 100 %.

Cet exemple montre la moins bonne activité de ce lot de catalyseur.

**EXEMPLE 4**

**PREPARATION DU CATALYSEUR :**

50 g. de l'alumine-$\gamma$ utilisée dans l'exemple 1 sont calcinés sous courant d'air à 350°C pendant 3 heures. Après refroidissement à 70°C, cette alumine est imprégnée par une solution aqueuse de 5,53 g. de perrhénate d'ammonium (teneur en rhénium théorique du catalyseur : 7,68 % en poids) dans 21 cm³ d'eau à 70°C en introduisant goutte-à-goutte la solution aqueuse à 70°C sur l'alumine et en agitant vigoureusement. On laisse ensuite l'alumine reposer en atmosphère humide pendant 20 heures à 70°C. Au bout de ce laps de temps, le solide est chauffé à une température de 140°C sous une pression de 0,1 mmHg pendant 2 heures. A ce moment, le catalyseur est remis sous pression atmosphérique d'air, puis balayé par un courant d'air contenant 90 ppm d'eau avec un débit de 25 l/h. La température est progressivement augmentée jusqu'à 550°C, niveau que l'on maintient pendant 3 heures. Le catalyseur ainsi activé est refroidi sous courant d'air à température ambiante, puis purgé plusieurs fois à l'azote et stocké sous azote.

**Utilisation en métathèse :**

17 g. du catalyseur préparé ci-dessus sont chargés dans l'appareillage décrit dans l'exemple 1. Du propylène est injecté liquide avec un débit de 71,4 cm³/h (VVH = 2,94), à une température de 70°C et sous une pression de 5 MPa. La conversion du propylène est de 39 % (sélectivité : pratiquement 100 %).

**EXEMPLE 5**

**PREPARATION DU CATALYSEUR :**

50 g. de l'alumine-$\gamma$ utilisée dans l'exemple 1 sont calcinés sous courant d'air à 350°C pendant 3 heures. Après refroidissement à température ambiante, cette alumine est imprégnée par une solution aqueuse d'acide perrhénique (21 cm³ contenant 3,85 g de rhénium métal, soit une teneur théorique en rhénium du catalyseur égale à 7,7 % poids) en introduisant goutte-à-goutte la solution aqueuse sur l'alumine et en agitant vigoureusement. On laisse ensuite l'alumine reposer en atmosphère humide pendant 20 heures à 20°C. Au bout de ce laps de temps, le solide, dont le poids n'a pas varié sensiblement, est porté en 15 minutes à une température de 120°C dans une étuve à circulation d'air, et maintenu dans ces conditions pendant 2 heures. A ce moment, le catalyseur est refroidi et transféré dans un four, où il est soumis à un balayage par un courant gazeux constitué par des gaz de combustion du méthane en présence d'un excès d'air, ce qui augmente progressivement la température jusqu'à 750°C, niveau que l'on maintient pendant 30 minutes. La composition de ce courant gazeux est la suivante : $CO_2$ = 4,58 ; $H_2O$ = 9,17 ; $O_2$ = 9,92 ; $N_2$ - 76,33 % volume. Le catalyseur ainsi activé est refroidi sous courant d'azote sec jusqu'à température ambiante, puis stocké sous azote.

**Utilisation en métathèse :**

17 g. du catalyseur préparé ci-dessus sont chargés dans l'appareillage décrit dans l'exemple 1. Du propylène liquide est injecté avec un débit de 107 cm$^3$/h (VVH = 4,40), à une température de 35° C et sous une pression de 4,5 MPa. La conversion du propylène est de 35 % (sélectivité : pratiquement 100 %).

**Revendications**

1. Procédé de préparation d'un catalyseur renfermant du rhénium, dans lequel on imprègne un support à base d'alumine par une solution d'un composé de rhénium dans un solvant, on chauffe ensuite à 85-250° C en éliminant le solvant d'imprégnation, et on chauffe finalement à 400-1000° C pour activer le catalyseur, caractérisé en ce que l'on imprègne le support à sec et en ce qu'on maintient le support imprégné, après la fin de l'addition de la solution du composé de rhénium, et avant le chauffage à 85-250° C, pendant au moins 10 heures à 0-80° C dans des conditions telles que au moins 85% du solvant adsorbé demeure dans le support imprégné, l'alumine utilisée ayant une porosité au moins égale à 0,1 cm$^3$/g et une surface au moins égale à 10 m$^2$/g

2. Procédé selon la revendication 1, caractérisé en ce que le rhénium, dans le catalyseur imprégné, est en proportion de 0.01 à 20% en poids du support.

3. Procédé selon la revendication 1 ou 2, dans lequel le composé de rhénium appartient au groupe constitué par l'heptoxyde de rhénium, le perrhénate d'ammonium et l'acide perrhénique.

4. Procédé selon l'une des revendications 1 à 3, dans lequel on chauffe à 85-250° C pendant 30 minutes à 5 heures en atmosphère neutre ou oxydante ou sous vide, puis on chauffe à 400-1000° C pendant 10 minutes à 5 heures en atmosphère non-réductrice.

5. Procédé selon l'une des revendications 1 à 4, dans lequel on chauffe à 600-1000° C pendant 10 minutes à 5 heures en atmosphère constituée par les gaz de combustion du mèthane en présence d'un excès d'air.

6. Catalyseur renfermant du rhénium, tel qu'obtenu par le procédé de l'une quelconque des revendications 1 à 5.

7. Utilisation du catalyseur du catalyseur renfermant du rhénium, tel qu'obtenu par le procédé de l'une quelconque des revendications 1 à 5, dans la production d'oléfines par métathèse.

**Claims**

1. A process for preparing a rhenium-containing catalyst, wherein an aluminacontaining carrier is impregnated with a solution of a rhenium compound in a solvent, then is heated to 85-250° C with removing of the impregnation solvent, and finally heated to 400-1000° C for activating the catalyst, characterized in that the carrier is dry-impregnated and the impregnated carrier is maintained after the end of the addition of the rhenium compound solution and before heating to 85-250° C for at least 10 hours at a temperature of 0-80° C under conditions such as at least 85 % of the adsorbed solvent remains in the impregnated carrier, the used alumina having a porosity of at least 0.1 cc/g and a surface of at least 10 m$^2$/g.

2. A catalyst according to claim 1, characterized by a rhenium content of the impregnated catalyst from 0.01 to 20 % by weight in proportion to the carrier.

3. A process according to claim 1 or 2, wherein the rhenium compound pertains to the group consisting of rhenium heptoxide, ammonium perrhenate and perrhenic acid.

4. A process according to one of claims 1 to 3, wherein the heating step at 85-250° C is conducted for 30 minutes-5 hours in neutral or oxidizing atmosphere or under vacuum, and followed with a heating at 400-1000− C for 10 minutes to 5 hours in non-reducing atmosphere.

5. A process according to one of claims 1 to 4, wherein the final heating step is conducted at 600-1000° C for 10 minutes-5 hours in atmosphere of gases formed by methane combustion in the presence of an air excess.

6. A rhenium-containing catalyst as obtained by the process according to any one of claims 1 to 5.

7. The use of a rhenium-containing catalyst, as obtained by the process of any one of claims 1 to 5, for olefin production by metathesis.

**Ansprüche**

1. verfahren zur Herstellung von Rhenium enthaltendem Katalysator, bei dem man einen Träger auf Aluminiumoxid-Basis mit einer Lösung eines Gemisches von Rhenium in einem Lösungsmittel imprägniert, dann auf 85 bis 250 °C erwärmt, indem man das Lösungsmittel der Imprägnierung entfernt wird, und schließlich zur Aktivierung des Katalysators auf 400 bis 1000 °C erwärmt, dadurch gekennzeichnet, daß man den Träger im Trockenen imprägniert und den imprägnierten Träger nach Abschluß der Zugabe der Lösung des Rhenium-Gemisches und vor der Erwärmung auf 85 bis 250 °C während mindestens 10 Stunden bei 0 bis 80 °C unter Bedingungen hält, bei denen mindestens 85 % des adsorbierten Lösungsmittels im imprägnierten Träger verbleiben, wobei das verwendete Alumiminiumoxid eine Porosität von mindestens etwa 0,1 cm$^3$/g und eine Oberfläche von mindestens etwa 10 m$^2$/g aufweist.

2. verfahren nach Anspruch 1, dadurch gekennzeichnet, daß das Rhenium im imprägnierten Katalysator im Umfang von 0,01 bis 20 Gew.-% des Trägers vorliegt.

3. verfahren nach Anspruch 1 oder 2, bei dem das Rhenium-Gemisch zu einer Gruppe aus Rheniumheptoxid, Ammoniumperrhenat und Perrheniumsäure gehört.

4. Verfahren nach einem der Ansprüche 1 bis 3, bei dem man während 30 Minuten bis 5 Stunden in normaler oder oxidierender Atmosphäre bzw. unter Vakuum auf 85 bis 250 °C erwärmt und dann während 10 Minuten bis 5 Stunden in nicht reduzierender Atmosphäre auf 400 bis 1000 °C erwärmt.

5. Verfahren nach einem der Ansprüche 1 bis 4, bei dem man während 10 Minuten bis 5 Stunden in einer sich aus Gasen der Verbrennung von Methan in Gegenwart von Luftüberschuß zusammensetzenden Atmosphäre auf 600 bis 1000 °C erwärmt.

6. Rhenium enthaltender Katalysator, erhältlich durch das Verfahren nach einem der Ansprüche 1 bis 5.

7. Verwendung des Rhenium enthaltenden, durch das Verfahren nach einem der Ansprüche 1 bis 5 erhältlichen Katalysators zur Herstellung von Olefinen durch Metathäsis.